# EUROPEAN PATENT APPLICATION

(11) **EP 4 837 911 A1**
(43) Date of publication of application: **11.11.2026**
(21) Application number: 24915174.7
(22) Date of filing: 26.12.2024
(51) Int. Cl.: C07D 495/04, A61K 31/5377, A61P 35/02, A61P 35/00

(54) **CRYSTAL FORMS OF THIENOPYRIMIDINE COMPOUND AND PREPARATION METHOD THEREFOR**

(30) Priority: 04.01.2024 CN 202410013431
(71) Applicant: BeBetter Med Inc., Guangzhou, Guangdong 510660 (CN)
(72) Inventor: HE, Jie, Guangzhou, Guangdong 510660 (CN); WANG, Kunlin, Guangzhou, Guangdong 510660 (CN); WANG, Li, Guangzhou, Guangdong 510660 (CN); CAI, Xiong, Guangzhou, Guangdong 510660 (CN)
(74) Representative: Fuchs Patentanwälte Partnerschaft mbB
(86) International application number: PCT/CN2024/142767
(87) International publication number: WO 2025/145959

(57) **Abstract**

Disclosed in the present invention are three crystal forms of thienopyrimidine compound A and a preparation method therefor, wherein crystal form I is of a trihydrochloride salt, and crystal form II and III are of dihydrochloride salts. The three crystal forms all have good stability and solubility, with a solubility of ≥4 mg/mL in a 5% aqueous glucose solution, so that the basic requirements for the solubility of a compound to be prepared into a lyophilized preparation for injection are met. The three crystal forms of thienopyrimidine compound A prepared in the present invention have good stability and solubility, can be prepared into a lyophilized preparation for clinical injection, and can be used for the effective treatment of patients with advanced, recurrent, etc. lymphoma, myeloma and lymphocytic leukemia, or drug-resistant patients.

## Description

The present invention claims the priority of a Chinese patent application filed with the China National Intellectual Property Administration on January 04, 2024, with application No. 2024100134315 and the title "Crystal Forms of Thienopyrimidine Compound and Preparation Method Therefor", the entire contents of which are incorporated herein by reference.

### Technical Field

The present invention belongs to the field of pharmaceutical technology, and particularly relates to crystal forms of thienopyrimidine compound and preparation method therefor.

### Background Art

The incidence of hematological malignancies is high. In the United States alone, there were approximately 184,720 newly diagnosed patients with hematological malignancies in 2023. New patients with leukemia, lymphoma and myeloma accounted for approximately 9.4% of the total newly diagnosed cancer cases in the United States in 2023. Among them, leukemia accounted for 32%, lymphoma accounted for 48%, and myeloma accounted for 19%. It is estimated that 1,629,474 people in the United States are suffering from hematological malignancies or are in recovery, and the disease types include leukemia, lymphoma, myeloma, myelodysplastic syndrome and myeloproliferative neoplasms (Leukemia and Lymphoma Society 2023). Statistics from the Chinese Anti-Cancer Association show that approximately 84,000 new lymphoma patients occur each year in China, with more than 47,000 deaths, and the number is increasing at a rate of 5% per year. In addition, in China, there are approximately 10,000-15,000 new myeloma patients each year, and more than 20,000 new cases of acute and chronic lymphocytic leukemia each year. At present, there is a lack of effective drugs for patients with advanced relapsed or drug-resistant lymphoma, myeloma and lymphocytic leukemia.

Phosphatidylinositol 3-kinase (PI3K) and histone deacetylase (HDAC) are important targets for the survival of tumor cells. HDAC inhibitors exert inhibitory effects on multiple tumor-cell messenger targets through epigenetic regulatory mechanisms. PI3K inhibitors and HDAC inhibitors have significant anticancer effects and have been clinically validated (Ho, T et al., Journal of Medicinal Chemistry 63, 12460-12484, 2020; Zhang, M et al., Chemical Science 11, 5855-5865, 2020; Vanhaesebroeck, B et al., Nature Reviews Drug Discovery 20, 741-769, 2021). Several known phosphatidylinositol 3-kinase inhibitors and histone deacetylase inhibitors, including Copanlisib, Alpelisib, Idelalisib, Vorinostat, Belinostat and the like, have been approved by the U.S. FDA for marketing; however, the above drugs cannot simultaneously inhibit phosphatidylinositol 3-kinase and histone deacetylase, and have poor efficacy for refractory or relapsed hematological malignancies, and thus cannot meet the increasing clinical demand.

A thienopyrimidine compound (compound A) is an HDAC (histone deacetylase)/PI3K (phosphatidylinositol 3-kinase) dual-target inhibitor, which selectively inhibits the synergistic tumor-cell messenger core protein kinase target PI3K and the epigenetic target HDAC, thereby disrupting the tumor-cell messenger network and exerting a strong killing effect on various tumor cells. In various hematological and solid xenograft tumor animal models, the inhibitor can strongly and effectively inhibit tumor growth, particularly exhibiting significant effects in various hematological B-cell malignancies, and safety evaluation experiments show that it has good safety. It can be used for effective treatment of patients with advanced relapsed lymphoma, myeloma and lymphocytic leukemia and the like, or drug-resistant patients. The structural formula thereof is as follows:

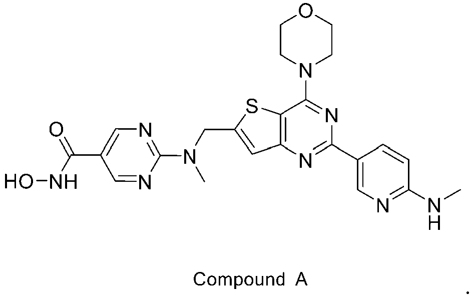

However, the compound has relatively low solubility, and when formulated into an injectable clinical formulation for use, the drug is prone to precipitate.

### Summary of the Invention

In view of the above, the objective of the present invention is to provide stable crystal forms of a thienopyrimidine compound, thereby improving the solubility thereof.

In order to achieve the above objective, the present invention provides the following technical solutions.

In a first aspect, the present invention provides a crystal form I of a thienopyrimidine compound. Expressed as 2θ, the crystal form I has characteristic peaks at 4.77°, 9.52°, 14.28°, 21.12°, 23.66°, 25.21° and 28.67° in an X-ray powder diffraction pattern, with an error of ±0.2°;
the structural formula of the thienopyrimidine compound is as follows:

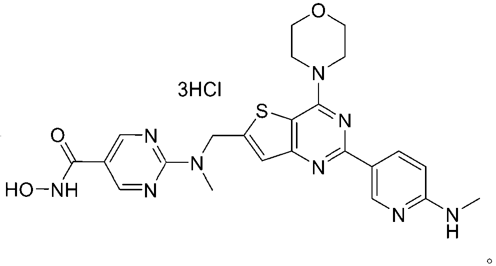

In some embodiments, expressed as 2θ, the crystal form I of the thienopyrimidine compound has characteristic peaks at 4.77°, 7.06°, 7.32°, 9.52°, 10.58°, 14.28°, 18.45°, 18.84°, 20.86°, 21.12°, 21.70°, 22.40°, 23.66°, 25.21°, 25.92°, 26.88°, 28.17°, 28.67°, 28.99°, 29.78°, 30.49° and 32.87° in an X-ray powder diffraction pattern, with an error of +0.2°.

In some embodiments, expressed as 2θ with an error of +0.2°, the characteristic peaks in the X-ray powder diffraction pattern of the crystal form I of the thienopyrimidine compound and the relative intensities of the characteristic peaks are as follows:

| 2 θ ° | Relative intensity |
|---|---|
| 4.77 | 25.80% |
| 7.06 | 19.30% |
| 7.32 | 14.40% |
| 9.52 | 83.30% |
| 10.58 | 11.30% |
| 11.20 | 8.00% |
| 14.28 | 42.50% |
| 15.14 | 6.20% |
| 16.92 | 6.80% |
| 17.70 | 6.10% |
| 18.45 | 14.20% |
| 18.84 | 19.00% |
| 20.86 | 11.10% |
| 21.12 | 26.60% |
| 21.70 | 17.90% |
| 22.40 | 12.00% |
| 23.26 | 6.70% |
| 23.66 | 64.00% |
| 25.21 | 100% |
| 25.92 | 15.50% |
| 26.88 | 17.70% |
| 28.17 | 14.90% |
| 28.67 | 32.00% |
| 28.99 | 18.70% |
| 29.78 | 15.00% |
| 30.49 | 15.30% |
| 31.41 | 6.70% |
| 32.87 | 13.10% |
| 35.69 | 4.10% |
| 37.25 | 4.00%. |

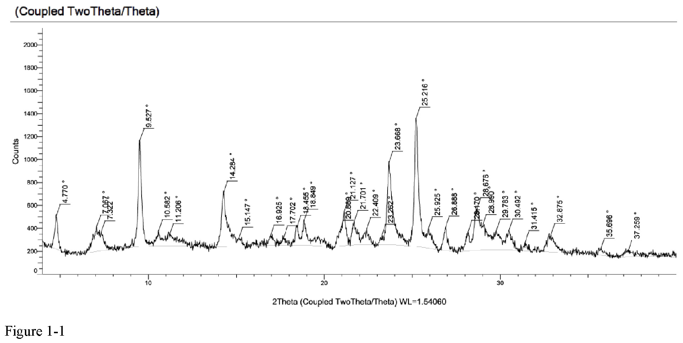

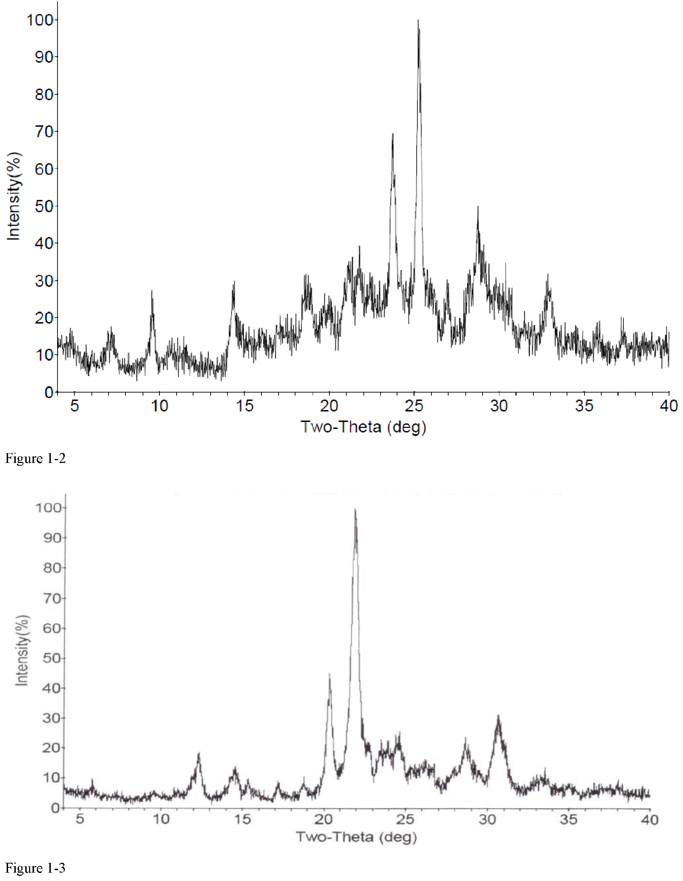

In some embodiments, the X-ray powder diffraction pattern of the crystal form I of the thienopyrimidine compound is as shown in Figure 1-1.

In some embodiments, a differential scanning calorimetry curve of the crystal form I of the thienopyrimidine compound comprises an exothermic peak at 220.9±0.5°C.

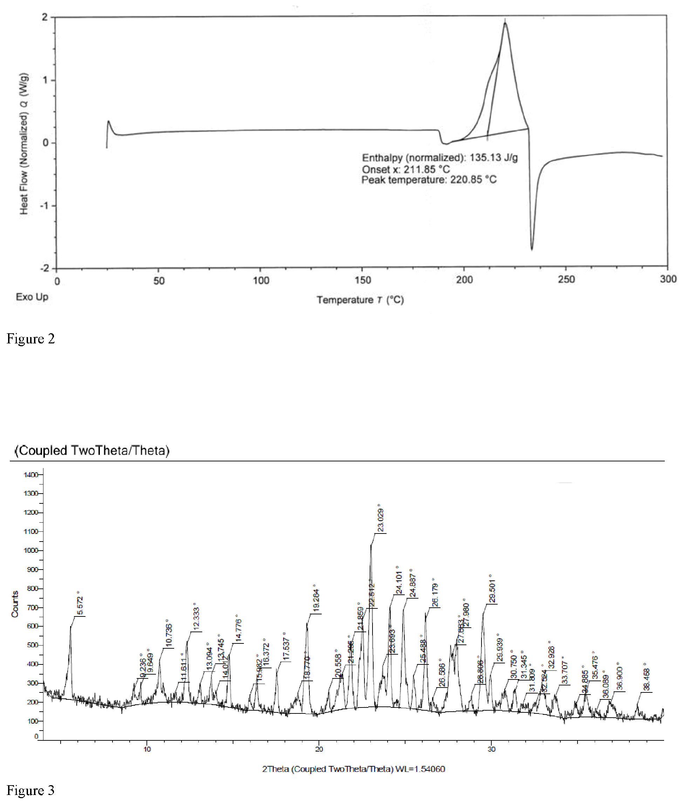

In some embodiments, the differential scanning calorimetry curve of the crystal form I of the thienopyrimidine compound is as shown in Figure 2.

In a second aspect, the present invention provides a crystal form II of a thienopyrimidine compound. Expressed as 2θ, the crystal form II has characteristic peaks at 5.57°, 19.28°, 21.85°, 22.51°, 23.02°, 24.10°, 24.88°, 26.17°, 27.98° and 29.50° in an X-ray powder diffraction pattern, with an error of +0.2°;
the structural formula of the thienopyrimidine compound is as follows:

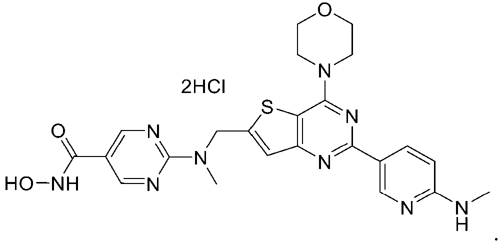

In some embodiments, expressed as 2θ, the crystal form II of the thienopyrimidine compound has characteristic peaks at 5.57°, 10.73°, 12.33°, 14.77°, 17.53°, 19.28°, 21.29°, 21.85°, 22.51°, 23.02°, 23.69°, 24.10°, 24.88°, 25.48°, 26.17°, 27.68°, 27.98°, 29.50° and 29.93° in an X-ray powder diffraction pattern, with an error of +0.2°. In some embodiments, expressed as 2θ with an error of +0.2°, the characteristic peaks in the X-ray powder diffraction pattern of the crystal form II of the thienopyrimidine compound and the relative intensities of the characteristic peaks are as follows:

| 2θ ° | Relative intensity |
|---|---|
| 5.57 | 44.00% |
| 9.23 | 9.00% |
| 9.64 | 11.20% |
| 10.73 | 26.00% |
| 11.61 | 4.80% |
| 12.33 | 36.20% |
| 13.09 | 12.00% |
| 13.74 | 17.90% |
| 14.01 | 7.60% |
| 14.77 | 31.80% |
| 15.98 | 9.00% |
| 16.37 | 19.00% |
| 17.53 | 23.70% |
| 18.77 | 12.70% |
| 19.28 | 54.40% |
| 20.55 | 13.00% |
| 21.29 | 20.20% |
| 21.85 | 40.10% |
| 22.51 | 54.00% |
| 23.02 | 100% |
| 23.69 | 25.00% |
| 24.10 | 61.80% |
| 24.88 | 60.50% |
| 25.48 | 20.40% |
| 26.17 | 56.20% |
| 26.58 | 7.10% |
| 27.68 | 34.10% |
| 27.98 | 44.30% |
| 28.80 | 8.80% |
| 29.50 | 58.70% |
| 29.93 | 22.00% |
| 30.75 | 11.70% |
| 31.34 | 11.90% |
| 31.80 | 4.20% |
| 32.52 | 4.40% |
| 32.92 | 19.70% |
| 33.70 | 11.70% |
| 34.88 | 6.70% |
| 35.47 | 15.20% |
| 36.08 | 3.50% |
| 36.90 | 8.70% |
| 38.45 | 8.9% . |

In some embodiments, the X-ray powder diffraction pattern of the crystal form II of the thienopyrimidine compound is as shown in Figure 3.

In some embodiments, a differential scanning calorimetry curve of the crystal form II of the thienopyrimidine compound comprises endothermic peaks at 79.5+0.5°C, 141.9+0.5°C and 207.7+0.5°C.

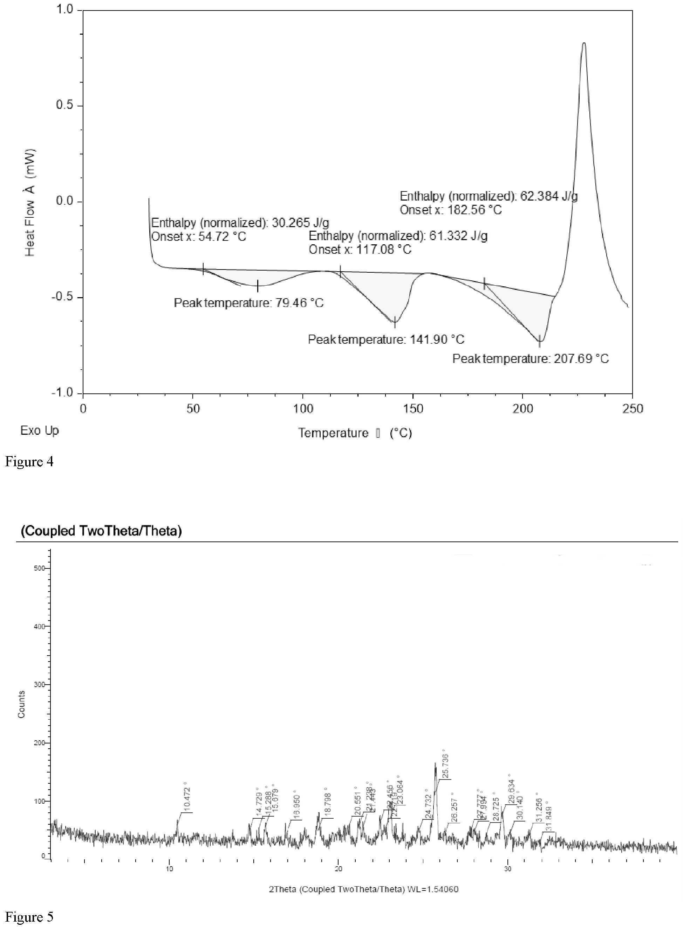

In some embodiments, the differential scanning calorimetry curve of the crystal form II of the thienopyrimidine compound is as shown in Figure 4.

In a third aspect, the present invention provides a crystal form III of a thienopyrimidine compound. Expressed as 2θ, the crystal form III has characteristic peaks at 10.47°, 15.67°, 21.23°, 21.44°, 22.45°, 23.06°, 25.73° and 29.63° in an X-ray powder diffraction pattern, with an error of +0.2°;
the structural formula of the thienopyrimidine compound is as follows:

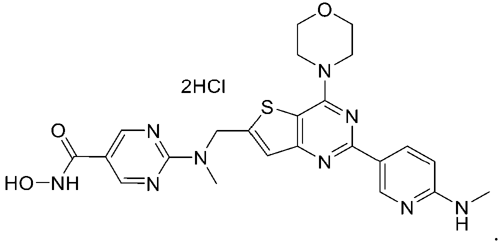

In some embodiments, expressed as 2θ, the crystal form III of the thienopyrimidine compound has characteristic peaks at 10.47°, 14.72°, 15.28°, 15.67°, 16.95°, 18.79°, 20.55°, 21.23°, 21.44°, 22.45°, 22.71°, 23.06°, 24.73°, 25.73°, 27.77°, 27.99°, 29.63°, 30.14° and 31.25° in an X-ray powder diffraction pattern, with an error of +0.2°. In some embodiments, expressed as 2θ with an error of +0.2°, the characteristic peaks in the X-ray powder diffraction pattern of the crystal form III of the thienopyrimidine compound and the relative intensities of the characteristic peaks are as follows:

| 2θ ° | Relative intensity |
|---|---|
| 10.47 | 32.80% |
| 14.72 | 24.80% |
| 15.28 | 28.10% |
| 15.67 | 32.40% |
| 16.95 | 22.30% |
| 18.79 | 24.90% |
| 20.55 | 26.20% |
| 21.23 | 35.20% |
| 21.44 | 31.40% |
| 22.45 | 37.70% |
| 22.71 | 23.50% |
| 23.06 | 48.10% |
| 24.73 | 23.10% |
| 25.73 | 100% |
| 26.25 | 15.90% |
| 27.77 | 26.00% |
| 27.99 | 22.00% |
| 28.72 | 17.70% |
| 29.63 | 52.00% |
| 30.14 | 21.30% |
| 31.25 | 21.60% |
| 31.84 | 9.20%. |

In some embodiments, the X-ray powder diffraction pattern of the crystal form III of the thienopyrimidine compound is as shown in Figure 5.

In some embodiments, a differential scanning calorimetry curve of the crystal form III of the thienopyrimidine compound comprises endothermic peaks at 96.1±0.5°C and 213.4+0.5°C.

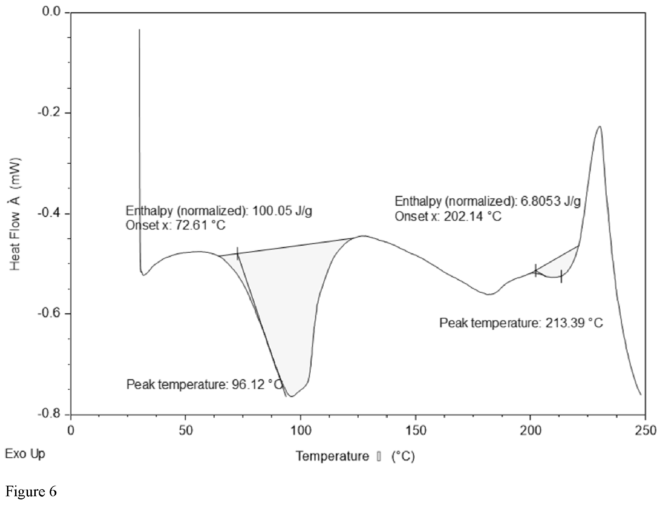

In some embodiments, the differential scanning calorimetry curve of the crystal form III of the thienopyrimidine compound is as shown in Figure 6.

In a fourth aspect, the present invention provides a method for preparing the crystal form I of the thienopyrimidine compound, comprising the following steps:
stirring a mixture of methanol, purified compound A and water until homogeneous, slowly adding a hydrogen chloride/methyl tert-butyl ether solution at a temperature of 0°C-10°C, then stirring at a temperature of 0°C-10°C for 15 hours-25 hours, filtering, washing and drying, thereby obtaining the crystal form I of the thienopyrimidine compound; wherein
the time for slowly adding the hydrogen chloride/methyl tert-butyl ether solution is 2 hours-3 hours;
the hydrogen chloride concentration in the hydrogen chloride/methyl tert-butyl ether solution is 4 mol/L-6 mol/L;
the structural formula of compound A is as follows:

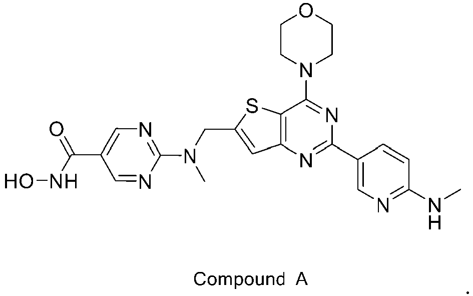

In some embodiments, the method for preparing the crystal form I of the thienopyrimidine compound comprises the following steps:
stirring a mixture of methanol, purified compound A and water at 20°C-30°C for 1.5 hours-2.5 hours, slowly adding a hydrogen chloride/methyl tert-butyl ether solution at a temperature of 4°C-6°C; then stirring at a temperature of 4°C-6°C for 18 hours-22 hours, filtering, washing and drying, thereby obtaining the crystal form I of the thienopyrimidine compound.

In some embodiments, the mass ratio of methanol to water is 100-130:1, preferably 105-125:1; preferably 110-120:1; preferably 114-118:1.

In some embodiments, the mass ratio of purified compound A to methanol is 1:11-14.

In some embodiments, the mass ratio of purified compound A to methanol is 1:12-13.

In some embodiments, the mass ratio of purified compound A to the hydrogen chloride/methyl tert-butyl ether solution is 1:2-5.

In some embodiments, the mass ratio of purified compound A to the hydrogen chloride/methyl tert-butyl ether solution is 1:3-4.

In some embodiments, the purification method of purified compound A comprises the following steps:
stirring dimethyl sulfoxide and compound A at a temperature of 45°C-55°C for 1.5 hours-2.5 hours, first adding methanol and compound A seed crystals at a temperature of 45°C-55°C, and stirring for 1.5 hours-2.5 hours, second adding methanol, and stirring for 1.5 hours-2.5 hours, cooling to 20°C-30°C, stirring for 1.5 hours-2.5 hours, filtering, washing and drying, thereby obtaining purified compound A.

In some embodiments, the purification method of purified compound A is as follows:
stirring dimethyl sulfoxide and compound A at a temperature of 48°C-52°C for 1.8 hours-2.2 hours. first adding methanol and compound A seed crystals at a temperature of 48°C-52°C, and stirring for 1.8 hours-2.2 hours, second adding methanol, and stirring for 1.8 hours-2.2 hours, cooling to 22°C-27°C, stirring for 1.8 hours-2.2 hours, filtering, washing and drying, thereby obtaining purified compound A.

In some embodiments, the mass ratio of dimethyl sulfoxide to compound A is 4-9:1, preferably 5-8:1; preferably 6-7:1.

In some embodiments, the mass ratio of methanol added in the first addition to compound A is 1-2:1.

In some embodiments, the mass of compound A seed crystals added is 0.4%-0.5% of the mass of compound A.

In some embodiments, the mass ratio of methanol added in the second addition to compound A is 12-20:1, preferably 14-18:1, preferably 15-17:1.

In a fifth aspect, the present invention provides a method for preparing the crystal form II of the thienopyrimidine compound, comprising the following steps:
adding the crystal form I of the thienopyrimidine compound to a mixed solution of ethanol and water, stirring at a temperature of 20°C-30°C for 72 hours-120 hours, filtering, washing and drying, thereby obtaining the crystal form II of the thienopyrimidine compound.

In some embodiments, the stirring time is 84 hours-108 hours, preferably 90 hours-102 hours, preferably 94 hours-98 hours, preferably 95 hours-97 hours.

In some embodiments, the volume ratio of ethanol to water is 2-4:1, preferably 2.5-3.5:1.

In some embodiments, the ratio of the mixed solution of ethanol and water to the crystal form I of the thienopyrimidine compound is 8-12 mL:1 g, preferably 9-11 mL:1 g.

In a sixth aspect, the present invention provides a method for preparing the crystal form III of the thienopyrimidine compound, comprising the following steps:
adding methanol to the crystal form I of the thienopyrimidine compound, stirring at a temperature of 20°C-30°C for 8 minutes-12 minutes, then stirring at a temperature of 45°C-55°C for 25 minutes-35 minutes, cooling to 0°C-10°C, and maintaining the temperature with stirring for 2 hours-4 hours, filtering, washing and drying, thereby obtaining the crystal form III of the thienopyrimidine compound.

In some embodiments, the method for preparing the crystal form III of the thienopyrimidine compound comprises the following steps:
adding methanol to the crystal form I of the thienopyrimidine compound, stirring at a temperature of 22°C-27°C for 9 minutes-11 minutes, then stirring at a temperature of 48°C-52°C for 28 minutes-32 minutes, cooling to 4°C-6°C, and maintaining the temperature with stirring for 2.5 hours-3.5 hours, filtering, washing and drying, thereby obtaining the crystal form III of the thienopyrimidine compound.

In some embodiments, the ratio of the crystal form I of the thienopyrimidine compound to methanol is 1 g:16-25 mL, preferably 1 g:18-22 mL, preferably 1 g:19-21 mL.

In some embodiments, the cooling rate for cooling to 0°C-10°C is 0.08-0.12°C/minute.

The present invention provides, through process optimization, three crystal forms of thienopyrimidine compound A, wherein crystal form I is a trihydrochloride, and crystal forms II and III are dihydrochlorides. The three crystal forms all have good stability and solubility, and have a solubility of ≥4 mg/mL in a 5% glucose aqueous solution, thereby meeting the basic solubility requirements for preparing an injectable lyophilized formulation. Among them, the solubilities of crystal form I and crystal form III in a 5% glucose aqueous solution are much higher than that of crystal form II, which can improve the dissolution rate, improve the storage stability of the drug solution prior to lyophilization of the injectable lyophilized formulation, and prevent drug precipitation after long-term standing, and are more suitable for being made into an injectable lyophilized formulation and are more preferred crystal forms.

The three crystal forms of thienopyrimidine compound A prepared in the present invention have good stability and solubility, and can be prepared into a clinical injectable lyophilized formulation for effective treatment of patients with advanced relapsed lymphoma, myeloma and lymphocytic leukemia and the like, or drug-resistant patients. Crystal form I of the present invention can be prepared from the free base of compound A in a methanol/water/hydrogen chloride/methyl tert-butyl ether solution system; crystal form II can be prepared from crystal form I by slurrying in EtOH/water; and crystal form III can be prepared from crystal form I by slow cooling in MeOH. The preparation processes of the three crystal forms are simple and suitable for industrial production.

### Brief Description of the Drawings

Figure 1-1 shows the XRPD pattern of crystal form I of compound (I) prepared by the method described in Example 1.
Figure 1-2 shows the XRPD pattern of crystal form I of compound (I) prepared by the method described in operation 1 of Comparative Example 1.
Figure 1-3 shows the XRPD pattern of a solid of compound (I) prepared by the method described in operation 2 of Comparative Example 1.
Figure 2 shows the DSC diagram of crystal form I of compound (I) prepared by the method described in Example 1.
Figure 3 shows the XRPD pattern of crystal form II of compound (I) prepared by the method described in Example 2.
Figure 4 shows the DSC diagram of crystal form II of compound (I) prepared by the method described in Example 2.
Figure 5 shows the XRPD pattern of crystal form III of compound (I) prepared by the method described in Example 3.
Figure 6 shows the DSC diagram of crystal form III of compound (I) prepared by the method described in Example 3.

### Detailed Description of the Disclosure

The technical solutions of the present invention are further described below through specific examples. Those skilled in the art should understand that the examples are only to help understand the present invention and should not be construed as specific limitations on the present invention.

Unless otherwise defined, all technical and scientific terms used herein have the meanings commonly understood by those skilled in the art in the technical field to which the present invention pertains. The terms used in the specification of the present invention are for the purpose of describing specific embodiments only and are not intended to limit the present invention.

The terms "comprise" and "have" and any variations thereof as used in the present invention are intended to cover non-exclusive inclusion. For example, a process, method, apparatus, product or device that comprises a series of steps is not limited to the listed steps or modules, but may optionally include steps that are not listed, or may optionally include other steps inherent to the process, method, product or device.

As used herein, "plurality" means two or more. "And/or", describing the association relationship of associated objects, indicates that three relationships may exist. For example, A and/or B may mean that A exists alone, A and B exist simultaneously, or B exists alone. The symbol "/" generally indicates that the associated objects before and after are in an "or" relationship.

The following are specific examples.

In the following examples, the operating parameters for X-ray powder diffraction (XRPD) analysis are set as follows:
Tube: Cu: K- Alpha (λ=1.54179Å).
Generator: Voltage: 40 kV; Current: 40 mA.
Scan Scope: 3 to 40 deg;
Sample rotation speed: 15 rpm.
Scanning rate: 10 deg/min or others.

The method used for differential scanning calorimetry (DSC) is as follows:
Under nitrogen protection at 50 mL/min, the sample in a pinholed aluminum pan is heated from 25°C to 250°C at 10 °C/min for testing.

The method for determining chloride ion content is as follows:
The chloride content of crystal form samples of compound (I) is determined by potentiometric titration according to General Rule <0701> of the Chinese Pharmacopoeia (2020 Edition), and is calculated based on the results corrected by weight content.

### Example 1. Preparation of crystal form I of compound (I)

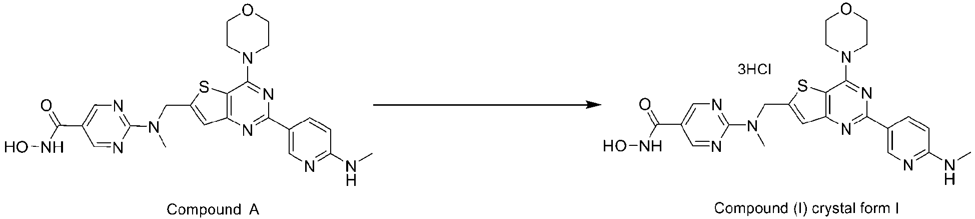

Compound (I) can be prepared from compound A. Compound A was prepared according to the synthesis method described in Chinese invention patent CN104292242B.

**Purification of compound** A: Dimethyl sulfoxide (56.8 kg) and compound A (8.7 kg) were charged into a reactor. The reactor temperature was adjusted to 50°C and the mixture was stirred for 2 hours until the materials were completely dissolved and the solution became clear. The solution was transferred to a crystallization vessel through an in-line filter. At 50°C, methanol (13 kg) and compound A seed crystals (0.04 kg) were added to the crystallization vessel, followed by stirring for 2 hours. Within 5 hours, methanol (139 kg) was added at a rate of not more than 30 kg/hour, followed by stirring for 2 hours. Within 5 hours, the temperature of the vessel was cooled to 25°C at 5°C/hour, followed by stirring for 2 hours. The resulting solid was collected by filtration. The solid was washed three times with methanol and dried to obtain purified compound A (8.39 kg).

**Preparation of crystal form I of compound (I):** Methanol (104.4 kg), the dried purified compound A obtained above (8.39 kg), and water for injection (0.9 kg) were charged into a reactor. The reactor temperature was adjusted to 25°C and the mixture was stirred for 2 hours. The reactor temperature was then adjusted to 5°C. A hydrogen chloride/methyl tert-butyl ether solution (5 mol/L, 31 kg) was slowly added (about 12 kg/hour), and the mixture was stirred at 5°C for 20 hours. The resulting solid was collected by filtration, washed three times with methyl tert-butyl ether, and dried to obtain a solid of crystal form I of compound (I) (9.14 kg), with a molar yield of 89.7% and a chloride content of 17%.

The XRPD pattern of crystal form I of compound (I) is shown in Figure 1-1, and the peak positions are listed in Table 1; the DSC diagram is shown in Figure 2.

**Table 1**

| Number | 2-Theta° | Relative intensity |
|---|---|---|
| 1 | 4.77 | 25.80% |
| 2 | 7.06 | 19.30% |
| 3 | 7.32 | 14.40% |
| 4 | 9.52 | 83.30% |
| 5 | 10.58 | 11.30% |
| 6 | 11.20 | 8.00% |
| 7 | 14.28 | 42.50% |
| 8 | 15.14 | 6.20% |
| 9 | 16.92 | 6.80% |
| 10 | 17.70 | 6.10% |
| 11 | 18.45 | 14.20% |
| 12 | 18.84 | 19.00% |
| 13 | 20.86 | 11.10% |
| 14 | 21.12 | 26.60% |
| 15 | 21.70 | 17.90% |
| 16 | 22.40 | 12.00% |
| 17 | 23.26 | 6.70% |
| 18 | 23.66 | 64.00% |
| 19 | 25.21 | 100% |
| 20 | 25.92 | 15.50% |
| 21 | 26.88 | 17.70% |
| 22 | 28.17 | 14.90% |
| 23 | 28.67 | 32.00% |
| 24 | 28.99 | 18.70% |
| 25 | 29.78 | 15.00% |
| 26 | 30.49 | 15.30% |
| 27 | 31.41 | 6.70% |
| 28 | 32.87 | 13.10% |
| 29 | 35.69 | 4.10% |
| 30 | 37.25 | 4.00% |

### Comparative Example 1. Preparation of crystal forms of compound (I) (crystal form not constant)

**Operation 1:** Purified compound A (8.78 kg) was suspended in methanol (126 kg) and dichloromethane (290 kg), and the suspension was cooled to 0-10°C. A 2.2 M hydrochloric acid/methanol solution (32 kg) was added at below 10°C. After the addition was completed, the mixture was stirred until the solid was completely dissolved, and then filtered. The filtrate was transferred to a clean room. Under stirring, methyl tert-butyl ether (140 kg) was added dropwise at 5-10°C. After the dropwise addition was completed, the mixture was further stirred at this temperature for 7 hours. After filtration, the obtained solid was rinsed with methyl tert-butyl ether (44 kg) to obtain a wet product (11.34 kg). The wet product was vacuum-dried at 40-50°C for 20 hours, and then vacuum-dried at 50-60°C for 30 hours until the water content was ≤5%, thereby obtaining a pale-yellow solid of compound (I) (10.04 kg, yield 94.1%, chloride content 16%). The XRPD pattern thereof is shown in Figure 1-2, which corresponds to crystal form I.

**Operation 2:** Purified compound A (600 g) was subjected to the preparation method of Operation 1 to obtain a pale-yellow solid of compound (I) (594 g, yield 78.2%, chloride content 16%). The XRPD pattern thereof is shown in Figure 1-3, which corresponds to an unknown crystal form.

The preparation methods of Example 1 and Comparative Example 1 were repeated, and multiple batches of crystal-form solids prepared therefrom were subjected to impurity-content testing and crystal-form identification. The impurity-content testing method is as follows:
A reverse-phase high-performance liquid chromatography (HPLC) method was used to determine the purity, content and related substances of compound I. The content was calculated by an external standard method, and the content of a single impurity was calculated by a main-component external standard method with correction factors.

### 1. Chromatographic conditions

Column: Agilent Zorbax SB-C18 (150 mm×4.6 mm, 3.5 µm); mobile phase A: trifluoroacetic acid-water (0.05:100); mobile phase B: trifluoroacetic acid-acetonitrile (0.05:100); detection wavelength: 250 nm; flow rate: 1.5 ml/min; injection volume: 5 µl; column temperature: 30°C; autosampler temperature: 15°C; run time: 50 min. Elution gradient:

| Time(min) | A% | B% |
|---|---|---|
| Start | 90 | 10 |
| 20.00 | 75 | 25 |
| 45.00 | 5 | 95 |
| 45.10 | 90 | 10 |
| 50.00 | 90 | 10 |

### Diluent:

Diluent 1: acetonitrile-water (50:50); Diluent 2: acetonitrile; Diluent 3: purified water.

Needle-washing solution: acetonitrile-water (50:50).

### 2. Preparation of solutions

(1) **Diluent 1 / blank solution:** acetonitrile-water (50:50): thoroughly mix 500 ml of acetonitrile with 500 ml of water. Label this solution as "BLK".
   Diluent 2: acetonitrile.
   Diluent 3: purified water.
(2) **Preparation of assay reference standard solutions** (compound I solution at 0.44 mg/ml):
   Prepare two solutions in parallel. Accurately weigh about 22 mg of the reference standard of compound I and place into a 50 ml volumetric flask. Add 25 ml of Diluent 2 to dissolve, sonicate for 5 minutes, allow to stand at room temperature, then dilute to volume with Diluent 3. Mix well, sonicate to dissolve, and cool to room temperature. Label as Reference Standard Solution-1 (A-STD-1) and Reference Standard Solution-2 (A-STD-2), respectively.
(3) **Preparation of related-substances reference standard solutions** (compound I solution at 0.022 mg/ml):
   Prepare two solutions in parallel. Pipette 5 ml of the assay reference standard solution (A-STD-1) into a 100 ml volumetric flask. Dissolve and dilute to volume with Diluent 1, and mix well. Label as Impurity Reference Standard Solution-1 (R-STD-1) and Impurity Reference Standard Solution-2 (R-STD-2), respectively.
(4) **Preparation of related-substances test solutions** (compound I solution at 2.2 mg/ml):
   Prepare two test solutions in parallel. Accurately weigh about 55 mg of the test sample and place into a 25 ml volumetric flask. Add 12.5 ml of Diluent 2 to dissolve, sonicate for 5 minutes, allow to stand at room temperature, then dilute to volume with Diluent 3. Mix well, sonicate to dissolve, and cool to room temperature. Label as Related-Substances Test Solution-1 (R-SPL-1) and Related-Substances Test Solution-2 (R-SPL-2), respectively.
(5) **Preparation of assay test solutions** (compound I solution at 0.44 mg/ml):
   Prepare two test solutions in parallel. Accurately pipette 5 ml of the test solution R-SPL-1 into a 25 ml volumetric flask. Dissolve and dilute to volume with Diluent 1, and mix well. Label as Assay Test Solution-1 (A-SPL-1) and Assay Test Solution-2 (A-SPL-2), respectively.

### 3. Calculation method

### Assay (content)

Calculate the content of the test sample according to the following formula: $Content \left(%, w/w\right) = \left(V_SPL\times A_SPL\right) / \left(W_SPL\times RF\right) \times 100 %$ where:
A_SPL represents the peak area of compound I in the test sample solution;
W_SPL represents the sample weight of compound I in the test sample solution (mg);
V_SPL represents the dilution volume of the test sample solution (ml);
RF represents the response factor of compound I.

Calculate the weight content of the free base of the test sample compound I according to the following formula: $Weight content of free base \left(%,w/w\right) = Content \left(%,w/w\right) \times M_F / M_S$ where:
A_SPL represents the peak area of compound I in the test sample solution;
W_SPL represents the sample weight of compound I in the test sample solution (mg);
V_SPL represents the dilution volume of the test sample solution (ml);
RF represents the response factor of compound I;
M_S represents the molecular weight of compound I, 616.95;
M_F represents the molecular weight of compound A, 507.57.

Calculate the content of the test sample on a dry basis according to the following formula: $Content \left(dry basis\right) \left(%, w/w\right) = \left(V_SPL\times A_SPL\right) / \left(W_SPL\times RF\times \left(100%-KF-RS\right)\right) \times 100 %$ where:
A_SPL represents the peak area of compound I in the test sample solution;
W_SPL represents the sample weight of compound I in the test sample solution (mg);
V_SPL represents the dilution volume of the test sample solution (ml);
RF represents the response factor of compound I;
KF represents the water content of the test sample of compound I (%);
RS represents the total residual solvents of the test sample of compound I (solvents with calculated results not less than the LOQ) (%).

Calculate the weight content of the free base of the test sample on a dry basis according to the following formula: 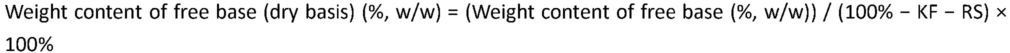 Weight content of free base (dry basis) (%, w/w) = (Weight content of free base (%, w/w)) / (100% - KF - RS) × 100% where:
A_SPL represents the peak area of compound I in the test sample solution;
W_SPL represents the sample weight of compound I in the test sample solution (mg);
V_SPL represents the dilution volume of the test sample solution (ml);
RF represents the response factor of compound I;
KF represents the water content of the test sample of compound I (%);
RS represents the total residual solvents of the test sample of compound I (solvents with calculated results not less than the LOQ) (%).

### (1) Impurity content

Compare the chromatogram of the test sample with the blank chromatogram, and integrate peaks with an area not less than 0.02% (LOD). The content of each individual impurity is calculated according to the following formula: $Individual impurity \left(%\right) = \left(V_S\times A_S\right) / \left(W_S\times RF_AVE\times RRF\right) \times 100 %$ where:
A_S represents the peak area of the impurity in the related-substances test sample solution;
W_S represents the weight of the related-substances test sample (mg);
RF_AVE represents the average response factor of compound I in six consecutive injections of the R-STD-1 solution;
V_S represents the dilution volume of the related-substances test sample solution (ml);
RRF represents the relative response factor of each impurity; the RRF of K-acid is 1.23, the RRF of K-ester is 1.16, and the RRF of other unspecified impurities is 1.00.

### (2) Total impurity content

Sum all individual impurities that are not less than 0.05%.

### (3) Purity

$Purity of compound I = 100 % - total impurities$

The comparison of the test results is shown in Table 2. The process of Comparative Example 1 involves adding a hydrochloric acid/methanol solution to a suspension of compound A in methanol/dichloromethane at 0-10oC, followed by dropwise addition of methyl tert-butyl ether to induce crystallization. With this process, the crystal form of the obtained compound is difficult to control; the crystal forms obtained from different batches are not constant, and the impurity content is relatively high. In the improved preparation process of Example 1, a hydrogen chloride/methyl tert-butyl ether solution is used to react with compound A and induce crystallization in the presence of methanol and water. This method can effectively control the impurity content and maintain consistent crystal forms across multiple batches.

**Table 2**

| | k-acid impurity% | k-ester impurity% | Total impurity | Crystal form |
|---|---|---|---|---|
| | 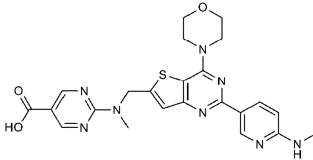 | 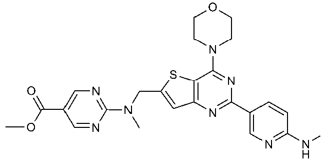 | | |
| Example 1 | 0.05-0.18% | 0.12-0.16% | 0.18-0.34 % | Form I |
| Comparativ e example 1 | 0.26-0.86% | 0.27-0.59% | 0.87-1.75 % | inconsisten t crystal form |

### Example 2. Preparation of crystal form II of compound (I)

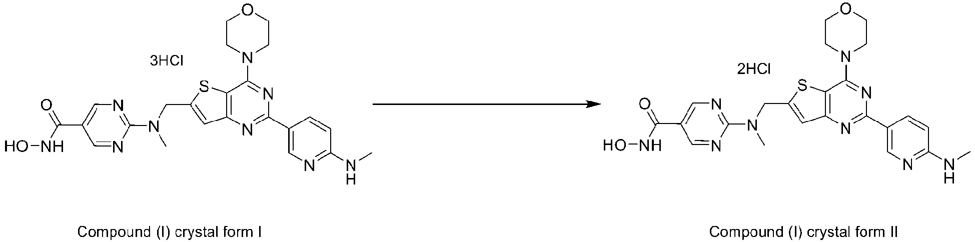

In a 2 L reactor, EtOH (900 mL) was charged, followed by addition of purified water (300 mL). Crystal form I of compound (I) (120 g) was added, and the mixture was stirred at 25°C for 96 hours. The mixture was then filtered, and the filter cake was rinsed with EtOH (200 mL). The product was vacuum-dried at 50°C for 20 hours to obtain a solid of crystal form II of compound (I) (108 g), with a molar yield of 90% and a chloride content of 12%.

The XRPD pattern of crystal form II of compound (I) is shown in Figure 3, and the peak positions are listed in Table 3; the DSC diagram is shown in Figure 4.

**Table 3**

| Number | 2-Theta° | Relative intensity |
|---|---|---|
| 1 | 5.57 | 44.00% |
| 2 | 9.23 | 9.00% |
| 3 | 9.64 | 11.20% |
| 4 | 10.73 | 26.00% |
| 5 | 11.61 | 4.80% |
| 6 | 12.33 | 36.20% |
| 7 | 13.09 | 12.00% |
| 8 | 13.74 | 17.90% |
| 9 | 14.01 | 7.60% |
| 10 | 14.77 | 31.80% |
| 11 | 15.98 | 9.00% |
| 12 | 16.37 | 19.00% |
| 13 | 17.53 | 23.70% |
| 14 | 18.77 | 12.70% |
| 15 | 19.28 | 54.40% |
| 16 | 20.55 | 13.00% |
| 17 | 21.29 | 20.20% |
| 18 | 21.85 | 40.10% |
| 19 | 22.51 | 54.00% |
| 20 | 23.02 | 100% |
| 21 | 23.69 | 25.00% |
| 22 | 24.10 | 61.80% |
| 23 | 24.88 | 60.50% |
| 24 | 25.48 | 20.40% |
| 25 | 26.17 | 56.20% |
| 26 | 26.58 | 7.10% |
| 27 | 27.68 | 34.10% |
| 28 | 27.98 | 44.30% |
| 29 | 28.80 | 8.80% |
| 30 | 29.50 | 58.70% |
| 31 | 29.93 | 22.00% |
| 32 | 30.75 | 11.70% |
| 33 | 31.34 | 11.90% |
| 34 | 31.80 | 4.20% |
| 35 | 32.52 | 4.40% |
| 36 | 32.92 | 19.70% |
| 37 | 33.70 | 11.70% |
| 38 | 34.88 | 6.70% |
| 39 | 35.47 | 15.20% |
| 40 | 36.08 | 3.50% |
| 41 | 36.90 | 8.70% |
| 42 | 38.45 | 8.9% |

### Example 3. Preparation of crystal form III of compound (I)

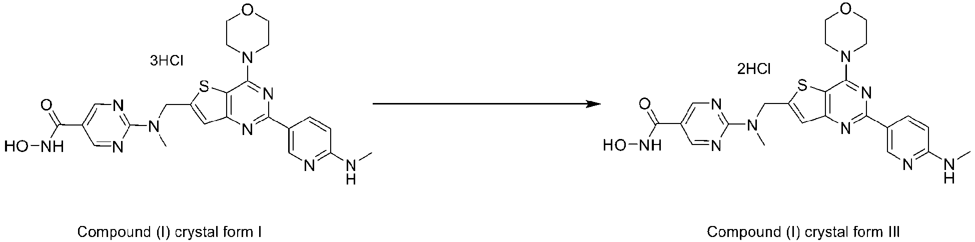

In a 500 ml reactor, crystal form I of compound (I) (10 g) and MeOH (200 ml) were charged. The mixture was stirred at 25°C for 10 minutes, and then stirred at 50°C for 30 minutes. The mixture was slowly cooled from 50°C to 5°C at 0.1°C/minute and maintained at this temperature with stirring for 3 hours. The mixture was filtered, and the filter cake was rinsed with MeOH (20 mL). The product was vacuum-dried at 50°C for 20 hours to obtain crystal form III of compound (I) (9.2 g), with a molar yield of 92% and a chloride content of 12%.

The XRPD pattern of crystal form III of compound (I) is shown in Figure 5, and the peak positions are listed in Table 4; the DSC diagram is shown in Figure 6.

**Table 4**

| Number | 2-Theta° | Relative intensity |
|---|---|---|
| 1 | 10.47 | 32.80% |
| 2 | 14.72 | 24.80% |
| 3 | 15.28 | 28.10% |
| 4 | 15.67 | 32.40% |
| 5 | 16.95 | 22.30% |
| 6 | 18.79 | 24.90% |
| 7 | 20.55 | 26.20% |
| 8 | 21.23 | 35.20% |
| 9 | 21.44 | 31.40% |
| 10 | 22.45 | 37.70% |
| 11 | 22.71 | 23.50% |
| 12 | 23.06 | 48.10% |
| 13 | 24.73 | 23.10% |
| 14 | 25.73 | 100% |
| 15 | 26.25 | 15.90% |
| 16 | 27.77 | 26.00% |
| 17 | 27.99 | 22.00% |
| 18 | 28.72 | 17.70% |
| 19 | 29.63 | 52.00% |
| 20 | 30.14 | 21.30% |
| 21 | 31.25 | 21.60% |
| 22 | 31.84 | 9.20% |

### Example 4. Comparison of chloride ion content and solubility of crystal forms I, II and III

The solubility determination method is as follows: 5 mg of the crystal form sample to be tested was weighed into an HPLC vial, and 1 mL of a 5% glucose solution was added. If the compound was substantially dissolved, an appropriate amount of additional compound solid was added; otherwise, the next step was carried out directly. A stir bar was added, and the mixture was stirred at 25 °C/700 rpm for 24 hours. The sample was transferred to a filtration centrifuge tube and centrifuged at 14000 rpm for 10 min. After 100-fold dilution, the sample was subjected to HPLC analysis to determine the sample content and calculate the solubility. The assay method is as described in Example 1.

The chloride ion content was determined by potentiometric titration according to General Rule <0701> of the Chinese Pharmacopoeia (2020 Edition) to determine the chloride content of the crystal form samples, and was calculated based on the results corrected by weight content. The method for determination of weight content is as described in Example 1.

The results are shown in Table 5. Crystal form I is a trihydrochloride, and crystal forms II and III are dihydrochlorides. All three crystal forms have a solubility of ≥4 mg/mL in a 5% glucose aqueous solution, thereby meeting the basic solubility requirements for preparing an injectable lyophilized formulation (minimum requirement: 4 mg/mL). If the solubility is low, it is likely to result in an excessively long dissolution time during preparation of the formulation solution, drug precipitation after long-term standing of the drug solution, or even incomplete dissolution, which is not conducive to the preparation of an injectable lyophilized formulation. Among the three crystal forms, the solubilities of crystal form I and crystal form III in a 5% glucose solution are much higher than that of crystal form II, making them more suitable for being made into an injectable lyophilized formulation, and thus they are more preferred crystal forms.

**Table 5**

| Crystal form | Chloride ion content (%) | Solubility (mg/ml) | Hydrochloride form |
|---|---|---|---|
| Form I | 17 | 9 | Trihydrochloride |
| Form II | 12 | 4 | Dihydrochloride |
| Form III | 12 | 7 | Dihydrochloride |

### Example 5. Stability of crystal form I sample of compound (I)

A sample of crystal form I of compound (I) was placed into a double-layer pharmaceutical low-density polyethylene bag, and each layer was separately sealed with a tie. A silica gel desiccant (product/desiccant: 5/1) was added, and the bag was placed into a single-layer aluminum foil bag for heat sealing. The sealed bag was then placed into a plastic drum. After being stored under different stability study conditions for a certain period of time, the impurity content of the sample was determined and compared with the test results of the Day 0 sample.

The experimental results are shown in Table 6, indicating that crystal form I of compound (I) has good stability.

**Table 6**

| | k-acid impurity% | k-ester impurity% | Total impurity% |
|---|---|---|---|
| Day 0 | 0.18 | 0.17 | 0.35 |
| 25°C/60% RH, 1M | 0.20 | 0.17 | 0.37 |
| 5°C±3, 1M | 0.18 | 0.17 | 0.35 |
| -20°C±5°C, 1M | 0.17 | 0.17 | 0.34 |

The technical features in the above examples may be combined in any manner. For the sake of brevity, all possible combinations of the technical features in the above examples are not described. However, as long as such combinations of technical features are not contradictory, they shall be deemed to fall within the scope of this specification.

The above examples merely set forth several embodiments of the present invention. Although the description is specific and detailed, it should not be construed as limiting the scope of the invention patent. It should be noted that, for those of ordinary skill in the art, various changes and improvements may be made without departing from the concept of the present invention, and such changes and improvements all fall within the protection scope of the present invention. Therefore, the protection scope of the present invention patent shall be determined by the appended claims.

## Claims

1. A crystal form I of a thienopyrimidine compound, **characterized in that**, expressed as 2θ, the crystal form I has characteristic peaks at 4.77°, 9.52°, 14.28°, 21.12°, 23.66°, 25.21° and 28.67° in an X-ray powder diffraction pattern, with an error of ±0.2°;
the structural formula of the thienopyrimidine compound is as follows:

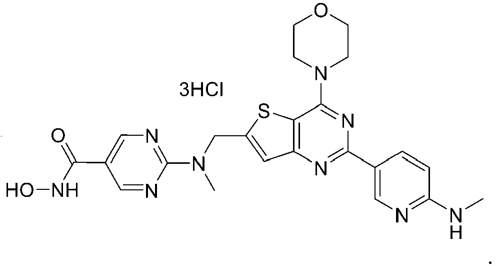

2. The crystal form I of the thienopyrimidine compound according to claim 1, **characterized in that**, expressed as 2θ, the crystal form I has characteristic peaks at 4.77°, 7.06°, 7.32°, 9.52°, 10.58°, 14.28°, 18.45°, 18.84°, 20.86°, 21.12°, 21.70°, 22.40°, 23.66°, 25.21°, 25.92°, 26.88°, 28.17°, 28.67°, 28.99°, 29.78°, 30.49° and 32.87° in an X-ray powder diffraction pattern, with an error of +0.2°.

3. The crystal form I of the thienopyrimidine compound according to claim 2, **characterized in that**, expressed as 2θ with an error of +0.2°, the characteristic peaks in the X-ray powder diffraction pattern and the relative intensities of the characteristic peaks are as follows:
| 2θ ° | Relative intensity |
|---|---|
| 4.77 | 25.80% |
| 7.06 | 19.30% |
| 7.32 | 14.40% |
| 9.52 | 83.30% |
| 10.58 | 11.30% |
| 11.20 | 8.00% |
| 14.28 | 42.50% |
| 15.14 | 6.20% |
| 16.92 | 6.80% |
| 17.70 | 6.10% |
| 18.45 | 14.20% |
| 18.84 | 19.00% |
| 20.86 | 11.10% |
| 21.12 | 26.60% |
| 21.70 | 17.90% |
| 22.40 | 12.00% |
| 23.26 | 6.70% |
| 23.66 | 64.00% |
| 25.21 | 100% |
| 25.92 | 15.50% |
| 26.88 | 17.70% |
| 28.17 | 14.90% |
| 28.67 | 32.00% |
| 28.99 | 18.70% |
| 29.78 | 15.00% |
| 30.49 | 15.30% |
| 31.41 | 6.70% |
| 32.87 | 13.10% |
| 35.69 | 4.10% |
| 37.25 | 4.00%. |

4. The crystal form I of the thienopyrimidine compound according to claim 1, **characterized in that** the X-ray powder diffraction pattern thereof is as shown in Figure 1-1.

5. The crystal form I of the thienopyrimidine compound according to any one of claims 1-4, **characterized in that** a differential scanning calorimetry curve thereof comprises an exothermic peak at 220.9+0.5°C.

6. The crystal form I of the thienopyrimidine compound according to claim 5, **characterized in that** the differential scanning calorimetry curve thereof is as shown in Figure 2.

7. A crystal form II of a thienopyrimidine compound, **characterized in that**, expressed as 2θ, the crystal form II has characteristic peaks at 5.57°, 19.28°, 21.85°, 22.51°, 23.02°, 24.10°, 24.88°, 26.17°, 27.98° and 29.50° in an X-ray powder diffraction pattern, with an error of ±0.2°;
the structural formula of the thienopyrimidine compound is as follows:

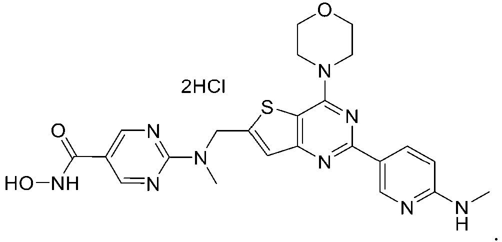

8. The crystal form II of the thienopyrimidine compound according to claim 7, **characterized in that**, expressed as 2θ, the crystal form II has characteristic peaks at 5.57°, 10.73°, 12.33°, 14.77°, 17.53°, 19.28°, 21.29°, 21.85°, 22.51°, 23.02°, 23.69°, 24.10°, 24.88°, 25.48°, 26.17°, 27.68°, 27.98°, 29.50° and 29.93° in an X-ray powder diffraction pattern, with an error of +0.2°.

9. The crystal form II of the thienopyrimidine compound according to claim 8, **characterized in that**, expressed as 2θ with an error of +0.2°, the characteristic peaks in the X-ray powder diffraction pattern and the relative intensities of the characteristic peaks are as follows:
| 2θ° | Relative intensity |
|---|---|
| 5.57 | 44.00% |
| 9.23 | 9.00% |
| 9.64 | 11.20% |
| 10.73 | 26.00% |
| 11.61 | 4.80% |
| 12.33 | 36.20% |
| 13.09 | 12.00% |
| 13.74 | 17.90% |
| 14.01 | 7.60% |
| 14.77 | 31.80% |
| 15.98 | 9.00% |
| 16.37 | 19.00% |
| 17.53 | 23.70% |
| 18.77 | 12.70% |
| 19.28 | 54.40% |
| 20.55 | 13.00% |
| 21.29 | 20.20% |
| 21.85 | 40.10% |
| 22.51 | 54.00% |
| 23.02 | 100% |
| 23.69 | 25.00% |
| 24.10 | 61.80% |
| 24.88 | 60.50% |
| 25.48 | 20.40% |
| 26.17 | 56.20% |
| 26.58 | 7.10% |
| 27.68 | 34.10% |
| 27.98 | 44.30% |
| 28.80 | 8.80% |
| 29.50 | 58.70% |
| 29.93 | 22.00% |
| 30.75 | 11.70% |
| 31.34 | 11.90% |
| 31.80 | 4.20% |
| 32.52 | 4.40% |
| 32.92 | 19.70% |
| 33.70 | 11.70% |
| 34.88 | 6.70% |
| 35.47 | 15.20% |
| 36.08 | 3.50% |
| 36.90 | 8.70% |
| 38.45 | 8.9% . |

10. The crystal form II of the thienopyrimidine compound according to claim 7, **characterized in that** the X-ray powder diffraction pattern thereof is as shown in Figure 3.

11. The crystal form II of the thienopyrimidine compound according to any one of claims 7-10, **characterized in that** a differential scanning calorimetry curve thereof comprises endothermic peaks at 79.5±0.5°C, 141.9±0.5°C and 207.7±0.5°C.

12. The crystal form II of the thienopyrimidine compound according to claim 11, **characterized in that** the differential scanning calorimetry curve thereof is as shown in Figure 4.

13. A crystal form III of a thienopyrimidine compound, **characterized in that**, expressed as 2θ, the crystal form III has characteristic peaks at 10.47°, 15.67°, 21.23°, 21.44°, 22.45°, 23.06°, 25.73° and 29.63° in an X-ray powder diffraction pattern, with an error of ±0.2°;
the structural formula of the thienopyrimidine compound is as follows:

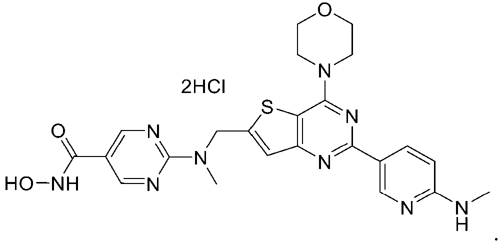

14. The crystal form III of the thienopyrimidine compound according to claim 13, **characterized in that**, expressed as 2θ, the crystal form III has characteristic peaks at 10.47°, 14.72°, 15.28°, 15.67°, 16.95°, 18.79°, 20.55°, 21.23°, 21.44°, 22.45°, 22.71°, 23.06°, 24.73°, 25.73°, 27.77°, 27.99°, 29.63°, 30.14° and 31.25° in an X-ray powder diffraction pattern, with an error of +0.2°.

15. The crystal form III of the thienopyrimidine compound according to claim 14, **characterized in that**, expressed as 2θ with an error of +0.2°, the characteristic peaks in the X-ray powder diffraction pattern and the relative intensities of the characteristic peaks are as follows:
| 2θ ° | Relative intensity |
|---|---|
| 10.47 | 32.80% |
| 14.72 | 24.80% |
| 15.28 | 28.10% |
| 15.67 | 32.40% |
| 16.95 | 22.30% |
| 18.79 | 24.90% |
| 20.55 | 26.20% |
| 21.23 | 35.20% |
| 21.44 | 31.40% |
| 22.45 | 37.70% |
| 22.71 | 23.50% |
| 23.06 | 48.10% |
| 24.73 | 23.10% |
| 25.73 | 100% |
| 26.25 | 15.90% |
| 27.77 | 26.00% |
| 27.99 | 22.00% |
| 28.72 | 17.70% |
| 29.63 | 52.00% |
| 30.14 | 21.30% |
| 31.25 | 21.60% |
| 31.84 | 9.20% . |

16. The crystal form III of the thienopyrimidine compound according to claim 13, **characterized in that** the X-ray powder diffraction pattern thereof is as shown in Figure 5.

17. The crystal form III of the thienopyrimidine compound according to any one of claims 13-16, **characterized in that** a differential scanning calorimetry curve thereof comprises endothermic peaks at 96.1±0.5°C and 213.4±0.5°C.

18. The crystal form III of the thienopyrimidine compound according to claim 17, **characterized in that** the differential scanning calorimetry curve thereof is as shown in Figure 6.

19. A method for preparing the crystal form I of the thienopyrimidine compound according to any one of claims 1-6, **characterized by** comprising the following steps:
stirring a mixture of methanol, purified compound A and water until homogeneous, slowly adding a hydrogen chloride/methyl tert-butyl ether solution at a temperature of 0°C-10°C, then stirring at a temperature of 0°C-10°C for 15 hours-25 hours, filtering, washing and drying, thereby obtaining the crystal form I of the thienopyrimidine compound;
wherein
the time for slowly adding the hydrogen chloride/methyl tert-butyl ether solution is 2 hours-3 hours;
the hydrogen chloride concentration in the hydrogen chloride/methyl tert-butyl ether solution is 4 mol/L-6 mol/L;
the structural formula of compound A is as follows:

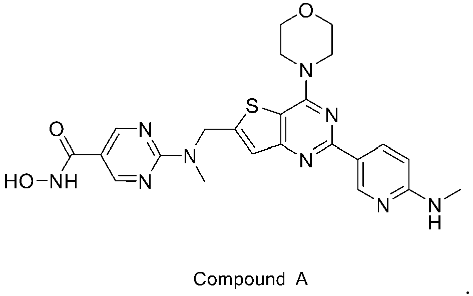

20. The method for preparing the crystal form I of the thienopyrimidine compound according to claim 19, **characterized by** comprising the following steps:
stirring a mixture of methanol, purified compound A and water at 20°C-30°C for 1.5 hours-2.5 hours, slowly adding a hydrogen chloride/methyl tert-butyl ether solution at a temperature of 4°C-6°C, then stirring at a temperature of 4°C-6°C for 18 hours-22 hours, filtering, washing and drying, thereby obtaining the crystal form I of the thienopyrimidine compound.

21. The method for preparing the crystal form I of the thienopyrimidine compound according to claim 19 or 20, **characterized in that** the mass ratio of methanol to water is 100-130:1, preferably 105-125:1; preferably 110-120:1; preferably 114-118:1; and/or,
the mass ratio of purified compound A to methanol is 1:11-14, preferably 1:12-13; and/or,
the mass ratio of purified compound A to the hydrogen chloride/methyl tert-butyl ether solution is 1:2-5, preferably 1:3-4.

22. The method for preparing the crystal form I of the thienopyrimidine compound according to claim 19 or 20, **characterized in that** the purification method of purified compound A comprises the following steps:
stirring dimethyl sulfoxide and compound A at a temperature of 45°C-55°C for 1.5 hours-2.5 hours, first adding methanol and compound A seed crystals at a temperature of 45°C-55°C, and stirring for 1.5 hours-2.5 hours, second adding methanol, and stirring for 1.5 hours-2.5 hours, cooling to 20°C-30°C, stirring for 1.5 hours-2.5 hours, filtering, washing and drying, thereby obtaining purified compound A.

23. The method for preparing the crystal form I of the thienopyrimidine compound according to claim 22, **characterized in that** the mass ratio of dimethyl sulfoxide to compound A is 4-9:1, preferably 5-8:1; preferably 6-7:1; and/or,
the mass ratio of methanol added in the first addition to compound A is 1-2:1; and/or,
the mass of compound A seed crystals added is 0.4%-0.5% of the mass of compound A; and/or,
the mass ratio of methanol added in the second addition to compound A is 12-20:1, preferably 14-18:1, preferably 15-17:1.

24. A method for preparing the crystal form II of the thienopyrimidine compound according to any one of claims 7-12, **characterized by** comprising the following steps:
adding the crystal form I of the thienopyrimidine compound to a mixed solution of ethanol and water, stirring at a temperature of 20°C-30°C for 72 hours-120 hours, filtering, washing and drying, thereby obtaining the crystal form II of the thienopyrimidine compound;
wherein the crystal form I of the thienopyrimidine compound is the crystal form I of the thienopyrimidine compound according to any one of claims 1-6.

25. The method for preparing the crystal form II of the thienopyrimidine compound according to claim 24, **characterized in that** the stirring time is 84 hours-108 hours, preferably 90 hours-102 hours, preferably 94 hours-98 hours, preferably 95 hours-97 hours; and/or,
the volume ratio of ethanol to water is 2-4:1, preferably 2.5-3.5:1; and/or,
the ratio of the mixed solution of ethanol and water to the crystal form I of the thienopyrimidine compound is 8-12 mL:1 g, preferably 9-11 mL:1 g.

26. A method for preparing the crystal form III of the thienopyrimidine compound according to any one of claims 13-18, **characterized by** comprising the following steps:
adding methanol to the crystal form I of the thienopyrimidine compound, stirring at a temperature of 20°C-30°C for 8 minutes-12 minutes, then stirring at a temperature of 45°C-55°C for 25 minutes-35 minutes, cooling to 0°C-10°C, and maintaining the temperature with stirring for 2 hours-4 hours, filtering, washing and drying, thereby obtaining the crystal form III of the thienopyrimidine compound;
wherein the crystal form I of the thienopyrimidine compound is the crystal form I of the thienopyrimidine compound according to any one of claims 1-6.

27. The method for preparing the crystal form III of the thienopyrimidine compound according to claim 26, **characterized in that** the ratio of the crystal form I of the thienopyrimidine compound to methanol is 1 g:16-25 mL, preferably 1 g:18-22 mL, preferably 1 g:19-21 mL; and/or,
the cooling rate for cooling to 0°C-10°C is 0.08-0.12°C/min.
